(19) Europäisches Patentamt
European Patent Office
Office européen des brevets

(11) EP 1 891 900 A1

(12) EUROPEAN PATENT APPLICATION
published in accordance with Art. 153(4) EPC

(43) Date of publication:
27.02.2008 Bulletin 2008/09

(51) Int Cl.:
*A61B 8/08* (2006.01)

(21) Application number: 06757007.7

(22) Date of filing: 06.06.2006

(86) International application number:
PCT/JP2006/311267

(87) International publication number:
WO 2006/132203 (14.12.2006 Gazette 2006/50)

(84) Designated Contracting States:
DE FR GB IT NL

(30) Priority: 07.06.2005 JP 2005166387

(71) Applicant: HITACHI MEDICAL CORPORATION
Tokyo (JP)

(72) Inventors:
• OSAKA, Takashi
-chome, Chiyoda-ku, Tokyo 1010047; (JP)
• MURAYAMA, Naoyuki
-chome, Chiyoda-ku, Tokyo 1010047; (JP)

(74) Representative: Strehl Schübel-Hopf & Partner
Maximilianstrasse 54
80538 München (DE)

(54) ULTRASONOGRAPHIC DEVICE AND ULTRASONIC ELASTIC IMAGE ACQUISITION METHOD

(57)    [Problem] To select a period suitable for acquiring an elastic image in accordance with variation of the displacement amount of a moving tissue when the elastic image of the moving tissue is acquired, thereby stably acquiring an elastic image of high image quality.

[Means for Resolution] A ultrasonic diagnostic apparatus including an ultrasonic probe for transmitting/receiving ultrasonic waves to/from a site containing a moving tissue of an examinee, ultrasonic wave transmitting means for outputting an ultrasonic wave signal for driving the ultrasonic probe, morphological image acquiring means for acquiring a morphological image representing morphological information of the site containing the moving tissue from a reflected echo signal received by the ultrasonic probe, elastic image acquiring means for acquiring an elastic image representing elastic information of the site containing the moving tissue by using a plurality of morphological image data, displacement amount detecting means for detecting a displacement amount of the moving tissue by using the morphological image data, and selecting means for selecting an elastic image acquiring period on the basis of the displacement amount, wherein the elastic image acquiring means acquires an elastic image during the selected period.

FIG.1

**Description**

Technical Field

The present invention relates to an ultrasonic diagnostic apparatus for acquiring a tomogram concerning a diagnostic site in an examinee by using ultrasonic waves, and particularly to a technique of acquiring an elastic image representing hardness or softness of the diagnostic site.

Background Art

A conventional general ultrasonic diagnostic apparatus is constructed by ultrasonic wave transmitting/receiving means for transmitting and receiving ultrasonic waves to/from an examinee, tomogram acquiring means for repetitively acquiring tomogram data in the examinee containing a moving tissue at a predetermined period by using a reflection echo signal received by the ultrasonic wave transmitting/receiving means, and image display means for displaying time-series tomograms acquired by the tomogram acquiring means. The ultrasonic diagnostic apparatus is configured to display morphological information of a biomedical tissue in the examinee as a B mode image (tomogram) or an M mode image on image display means.
It has been recently proposed in the conventional general ultrasonic diagnostic apparatus that strain or elasticity is measured by using plural tomograms of a biomedical tissue of a diagnostic site, and also an elastic image based on the measurement result is displayed on image display means. A technique disclosed in the following patent document 1 is known as an ultrasonic diagnostic apparatus configured to display an elastic image.
The patent document 1 discloses an example in which an elastic image of a blood vessel wall is acquired by using the fact that the blood vessel wall itself moves without applying pressure from the external thereto due to repetitive expansion and contraction of the blood vessel in accordance with a periodical variation of the pressure in the blood vessel of carotid artery which is caused by pulsation of a heart. Particularly, the elasticity of the blood vessel wall is calculated by using the maximum value of the strain amount. Furthermore, a stiffness parameter as one kind of an elastic coefficient is calculated by using the maximum and minimum values of the diameter of the artery.
Patent Document 1: JP-A-2000-229078
In general, in order to acquire an elastic image from plural tomograms stably, it is required that the displacement amount of the biomedical tissue is equal to some degree or more among the plural tomograms. This is because the displacement amount can be stably detected when the displacement amount of the biomedical tissue is large.
The pressure variation in the blood vessel is derived from the pulsation of the heart, and the pressure in the blood vessel rapidly increases and the blood vessel sharply expands during the period when blood is strongly ejected from the heart into the artery. Therefore, the displacement of the blood vessel wall is also quick, and thus this period is suitable to acquire an elastic image of the blood vessel wall.
However, as disclosed in the Patent Document 1, according to the method for obtaining elasticity information by using the maximum value of the strain amount and the maximum and minimum values of the diameter of the artery, thereby imaging elastic images, the elastic images of the blood vessel wall are at most intermittently obtained, and valuable image information is not sufficiently actively used for diagnosis.

Disclosure of the Invention

The present invention has an object to provide an ultrasonic diagnostic apparatus and an elastic image acquiring method that can stably acquire an elastic image of high image quality in accordance with a displacement amount of a moving tissue when the elastic image of the moving tissue is acquired.
In order to attain the above object, the ultrasonic diagnostic apparatus of the present invention is constructed as follows. That is, the ultrasonic diagnostic apparatus is characterized by comprising: an ultrasonic probe for transmitting/receiving ultrasonic waves to/from a site containing a moving tissue of an examinee; ultrasonic wave transmitting means for outputting an ultrasonic wave signal for driving the ultrasonic probe; morphological image acquiring means for acquiring a morphological image representing morphological information of the site containing the moving tissue from a reflected echo signal received by the ultrasonic probe; elastic image acquiring means for acquiring an elastic image representing elastic information of the site containing the moving tissue by using a plurality of morphological image data; displacement amount detecting means for detecting a displacement amount of the moving tissue by using the morphological image data; and selecting means for selecting an elastic image acquiring period on the basis of the displacement amount, wherein the elastic image acquiring means acquires an elastic image during the selected period.
Furthermore, in order to attain the above object, a method of acquiring an ultrasonic elastic image is constructed as follows. That is, a method of acquiring an ultrasonic elastic image of a site containing a moving tissue of an examinee is characterized by comprising:

(a) a step of acquiring a morphological image representing morphological information of the site containing the moving tissue;

(b) a step of acquiring an elastic image representing elastic information of the site containing the moving tissue by using a plurality of morphological image data; and

(c) a step of repeating the steps (a) and (b), wherein in the elastic image acquiring step, a displacement amount of the moving tissue is detected by using the morphological image data, and an elastic image acquiring period is selected on the basis of the displacement amount.

According to the present invention, when the elastic image of the moving tissue is acquired, the acquisition of the elastic image is controlled in accordance with the displacement amount of the moving tissue, whereby an elastic image having high image quality can be stably acquired.

Best Mode for carrying out the Invention

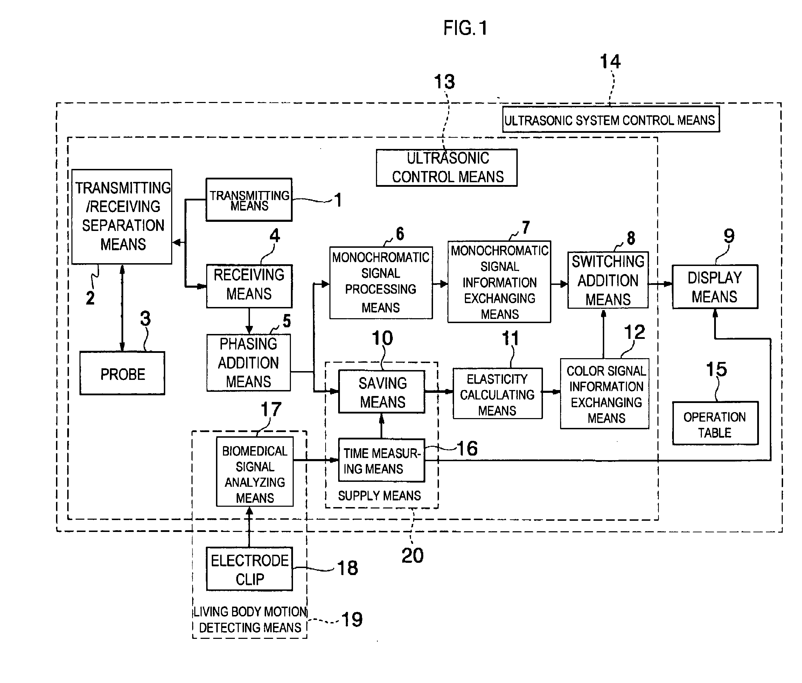

An embodiment of an ultrasonic diagnostic apparatus according to the present invention will be hereunder described with reference to the drawings. Fig. 1 is a block diagram showing an embodiment of the ultrasonic diagnostic apparatus according to the present invention.

The ultrasonic diagnostic apparatus according to the present invention comprises transmitting means 1 for generating ultrasonic waves to be transmitted to an examinee, a probe 3 for transmitting/receiving ultrasonic waves while the probe is brought into contact with the examinee, receiving means 4 for amplifying a reflected echo signal of the ultrasonic waves transmitted from the probe 3, transmission/reception separating means 2 for switching transmission and reception of the ultrasonic waves, phasing addition means 5 for subjecting the received reflected echo signal to phasing addition, monochromatic signal processing means 6 for calculating signal intensity of a biomedical tissue with respect to an output signal of the phasing addition means 5, monochromatic signal information converting means 7 for constructing an ultrasonic monochromatic tomogram, saving means 10 for saving plural frames corresponding to the output signal of the phasing addition means 5 on a frame basis, elasticity calculating means 11 for measuring the displacement amount of the biomedical tissue from RF frame data corresponding to the output signal from the saving means 10 to calculate elasticity or strain, color signal information exchanging means 12 for constructing ultrasonic elastic image data corresponding to the output signal of the elasticity calculating means 11 as a color image, switching addition means 8 for superposing the output signals of the monochromatic signal information converting means 7 and the color signal information exchanging means 12 and displaying the superposition signal, biomedical signal analyzing means 17 for processing a biomedical signal detected by using an electrode clip 18 for detecting an electrocardiogram of the examinee, time measuring means 16 for measuring a time difference from any reference time point in an electrocardiographic wave, and ultrasonic control means 13 for controlling the respective means to control transmission/reception of ultrasonic waves.

Furthermore, the ultrasonic diagnostic apparatus according to the present invention further comprises display means 9 for displaying superposed ultrasonic monochromatic tomogram and ultrasonic elastic image, an operating table 15 for executing various kinds of operations of the ultrasonic diagnostic apparatus such as various measurements, mode switching, etc., and ultrasonic system control means 14 for controlling the overall system of the ultrasonic diagnostic apparatus on the basis of control information input through the operation table 15.

As not shown, the ultrasonic diagnostic apparatus of the present invention is equipped with a pressure sensor (not shown) for measuring the pressure applied to the examinee. Each of the means described above will be described hereunder with reference to Figs. 1 to 15.

First, transmission/reception of ultrasonic waves which is generally known in the ultrasonic diagnostic apparatus will be described.

The transmitting means 1 has the function of generating a transmission wave pulse for driving the probe 3 to generate ultrasonic waves and also setting the convergent point of the transmitted ultrasonic waves to some depth. The probe 3 is formed by disposing plural ultrasonic oscillators. The probe 3 has the function of carrying out beam scanning electrically to transmit/receive ultrasonic waves to/from an examinee through the oscillators. The receiving means 4 has the function of amplifying a reflected echo signal received by the probe 3 at a predetermined gain to generate an ultrasonic signal. The phasing addition means 5 has the function of receiving the ultrasonic reception signal amplified by the receiving means 4 and then executing phase control on the received signal. The phasing addition means 5 has the function of forming ultrasonic beams converged to plural convergent points and generating an RF signal as ultrasonic raw data.

Next, the construction of the ultrasonic monochromatic tomogram will be described.

The monochromatic signal processing means 6 has the function of receiving the RF signal from the phasing addition means 5 and executing various kinds of signal processing such as gain correction/log compression/wave detection/edge enhancement/filter processing, etc. on the RF signal to acquire ultrasonic monochromatic tomogram data. The monochromatic signal information exchanging means 7 is a so-called scan converter, and it is constructed by an A/D converter (not shown) for converting tomogram data from the monochromatic signal processing means 6 to a digital signal, a frame memory (not shown) for storing plural converted tomogram data in time-series style, and a control controller (not shown).

The monochromatic signal information exchanging means 7 has the function for acquiring tomographic RF frame data of the examinee stored in the frame memory as one image and reading out the acquired tomographic RF frame data under TV-synchronization. The data read out in the monochromatic signal information exchanging means 7 is displayed on the display means 9 through the switching addition means 8.

The switching addition means 8 has the function of determining an addition rate of an ultrasonic monochromatic tomogram constructed by the monochromatic signal information exchanging means 7 and an ultrasonic elastic image described later when the ultrasonic monochromatic tomogram and the ultrasonic elastic image are superposed on (combined with) each other. An addition method will be descried. In general, when an addition coefficient is represented by $\alpha$, it is determined by the following equation.

$$\texttt{Pix\_Sum(i,j)=}\alpha \bullet \texttt{Pix\_B(i,j)+(1-}\alpha \texttt{)} \bullet \texttt{Pix\_S(i,j)} \qquad (1)$$

Here, (i,j) represents the coordinate of a pixel, Pix_Sum (i,j) represents the pixel value after superposition, Pix_B(i,j) represents the pixel value of a monochromatic tomogram, and Pix_S(i,j) represents the pixel value of an elastic image. The tomogram image and the elastic image may be separately displayed without being superposed.

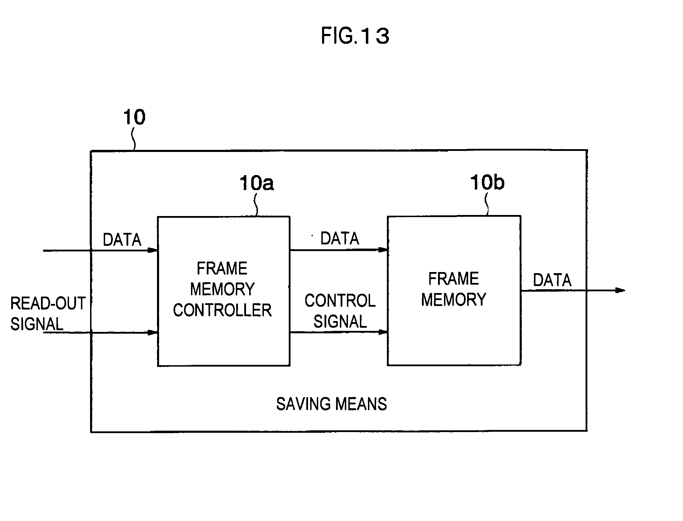

As shown in Fig. 13, the saving means 10 comprises a frame memory 10b for storing the RF signal from the phasing addition means 5 by the amount corresponding to plural frames, and a frame memory controller 10a for controlling writing/reading into/from the frame memory 10b. The output from the phasing addition means 5 is continuously carried out while ultrasonic waves are transmitted/received. The frame memory controller 10a has the function of controlling the frame memory 10b to time-sequentially repeat saving and renewal of RF frame data (which is an RF signal data group of one frame and equivalent to tomogram data). As described later, when time differences t1, t2 are measured in the time measuring means 16, a read-out signal of data is output from the time measuring means 16 to the saving means 10, and on the basis of the this data read-out signal, the frame memory controller 10a outputs a read-out signal to the frame memory 10b to transfer the RF frame data to the elasticity calculating means 11 at the rear stage.

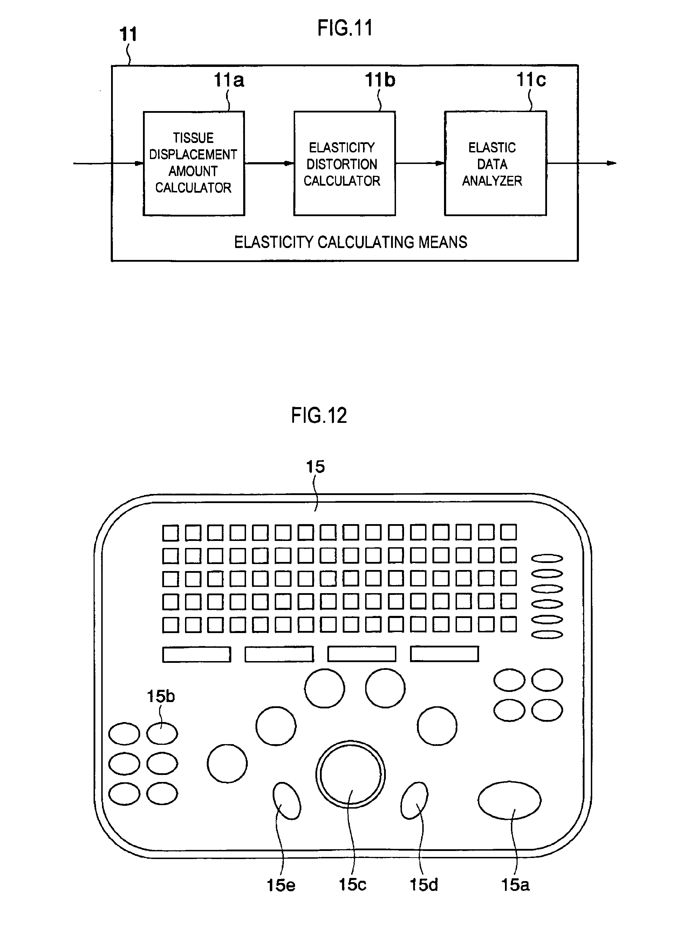

As shown in Fig. 11, the elasticity calculating means 11 is constructed by a tissue displacement amount calculator 11a, an elasticity/strain calculator 11b and an elasticity data analyzer 11c. The tissue displacement amount calculator 11a has the function of calculating the displacement amount of a biochemical tissue from one set of RF frame data output from the saving means 10 on the basis of the read-out signal.

For example, one-dimensional or two-dimensional correlation processing is executed from RF frame data (N) and RF frame data (X) to obtain the displacement and moving vector of the biomedical tissue corresponding to each point of the tomogram, that is, a one-dimensional or two-dimensional displacement distribution concerning the direction and degree of the displacement. Here, the moving vector is detected by using a block matching method. According to the block matching method, an image is divided into blocks each of which includes K X L pixels, and attention is paid to a block in an interested area. A block which is closest to the interested block is searched from another RF frame data, and prediction coding is executed by referring the searched block. That is, the processing of determining a sample value on the basis of the difference is executed.

The elasticity/strain calculator 11b has the function of calculating elasticity/strain on the basis of the one-dimensional or two-dimensional displacement distribution calculated by the tissue displacement amount calculator 11a. For example, when the displacement calculated by the tissue displacement amount calculator 11a is represented by $\Delta L$ and the pressure measured by a pressure sensor (not shown) which can measure the pressure applied to an examinee is represented by $\Delta P$, the strain is calculated by spatially differentiating $\Delta L$ ($\Delta L / \Delta X$). Out of the elasticity, Young's modulus Ym is calculated according to the equation: Ym = $(\Delta P)/(\Delta L/L)$. Young's modulus means the ratio between the tensile stress applied to an object and strain occurring in parallel to the tension, and represents the physical absolute value. Elastic image data representing the two-dimensional distribution of the strain and the elasticity (Young's modulus Ym) thus determined is achieved.

The elasticity data analyzer 11c has the function of executing the processing for enabling proper elasticity data to be stably displayed on the display means 9 on the basis of the elasticity data calculated in the elasticity/strain calculator 11b. An example of the stabilizing processing in the elasticity data analyzer 11c will be described. That is, a method of generating proper elastic RF frame data will be described.

It is generally known that an excellent strain image is achieved when pressurization or depressurization is applied in one set of RF frame data to the extent that 1% strain occurs. In this invention, plural sets of RF frame data are mixed with RF frame data in which only a displacement providing a calculated strain average value less than a half of 1% (for example, this value is set as a threshold value) is applied, the RF frame data concerned is rejected. Accordingly, an elastic image calculated by using only RF frame data to which a proper press amount is added is displayed on the display means 9. As another stabilizing method, smoothing processing is executed on continuously calculated elasticity data in the time direction, whereby the linkage of continuously displayed frames is improved.

The color signal information exchanging means 12 has the function of converting the elasticity data from the elasticity data analyzer 11c to hue information. That is, it has the function of converting the elasticity data to three primary colors of light, that is, red (R), green (G) and blue (B) on the basis of the elasticity data. For example, elastic data having small strain is converted to a blue code at the same time when elastic data having large strain are converted to a red code. Red (R), Green (G) and Blue(B) are assumed to have 256 gradations, and 255 is assumed to represent large brightness. Conversely, 0 is assumed to mean no display.

Next, the control of the ultrasonic diagnostic apparatus will be described.

The ultrasonic control means 13 executes the control associated with transmission/reception of ultrasonic waves, and has the function of executing the essential control of the ultrasonic diagnostic apparatus such as the control of wave transmission timing in each mode, the sequence control, distribution of synchronization signals and clocks to each signal processing means, etc. In Fig. 1, illustration of lines representing the flow of control signals from the ultrasonic control means 13 to other means is omitted.

The ultrasonic system control means 14, the display means 9 and the operating table 15 have the function of a so-called personal computer. Specifically, it has the function of controlling the overall system of the ultrasonic diagnostic apparatus, that is, the function of controlling a part constructed by hardware and a part controlled by software. In Fig. 1, illustration of lines representing the flow of control signals from the ultrasonic system control means 14 to the other respective means is omitted.

The display means 9 has the monitor function of displaying ultrasonic images such as a monochromatic tomogram (B mode image), an M mode image, an elastic image, etc. The operating table 15 has the function of playing a role like a keyboard for executing various kinds of operations. As shown in Fig. 12, the operating table 15 is provided with a freeze button 15a, a caliper start button 15b, a track ball 15c, an enter button 15d, an automatic measuring button 15e, etc.

(First Embodiment)

Next, a first embodiment of the present invention will be described. In this embodiment, selection of the elastic image acquiring period and control of the elastic image acquiring frequency are performed in accordance with the displacement of a blood vessel wall which is caused by heart pulsation. The displacement of the blood vessel wall is detected by using an M mode image, for example. Firstly, a time measuring means 16 and a biomedical signal analyzing means 17 constituting a living body motion detecting means 19 will be described.

The biomedical signal analyzing means 17 has the function of taking in a biomedical signal occurring in an examinee and displaying it as an electrocardiogram on the display means 9. In general, when an electrocardiogram is measured, an electrode clip 19 is mounted on each of a wrist and an ankle of the examinee for measurement, and an electrical exciting state occurring between the superior aorta called as an auricular node of cardiac muscle and the right atrium is recorded as a potential difference (voltage) variation.

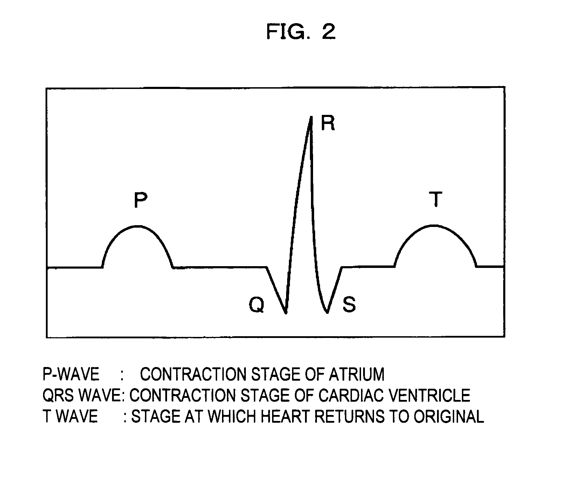

Fig. 2 shows a generally measured electrocardiogram. Respective places on the electrocardiogram shown in Fig. 2 are named as P, Q, R, S, T, and they represent the following operations of the heart. That is, they are discriminated from one another so that P wave represents the contraction stage of the atrium, QRS wave represents the contraction stage of the ventricle, and T wave represents the state under which the heart is returned to the original state.

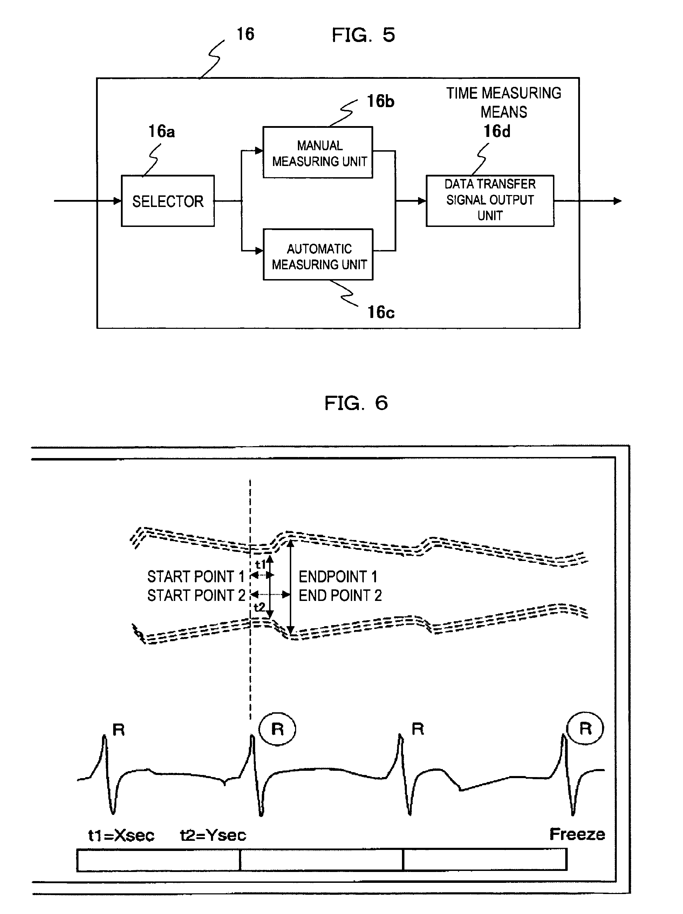

The time measuring means 16 is connected to the biomedical signal analyzing means 17 so that an electrocardiographic wave from the biomedical signal analyzing means 17 is input to the time measuring means 16, and has the function of detecting an R time point from the input electrocardiographic wave. Furthermore, the time measuring means 16 outputs the data read-out signal from the data transfer signal output portion 16d to the saving means 10 so that the RF frame data at the time point having a desired time difference from the detected R time point are transferred from the saving means 10 to the elasticity calculating means 11. In this case, the data read-out signal is not output at the R time point which appears every time, but the data read-out signal is assumed to be output at each in-circle R (a symbol of R surrounded by a circle in Fig. 6) time point which is located at every other R time point.

Next, the electrocardiogram and the blood vessel wall will be described.

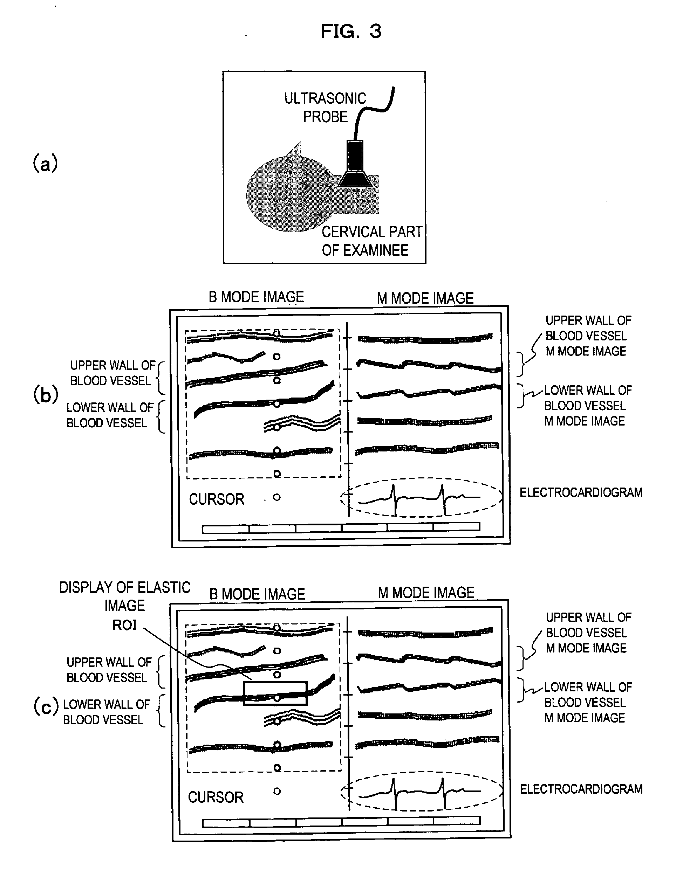

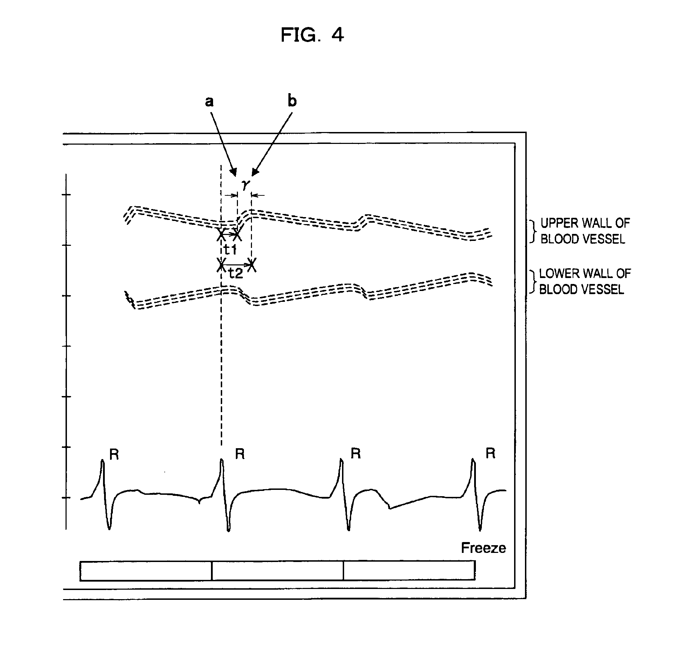

Fig. 3 is a schematic diagram of an ultrasonic image when the probe 3 is brought into contact with the carotid artery of the examinee as shown in (a) and an ultrasonic tomogram (B mode image, at the left side of (b)), an M mode image (at the right side of (b)) and electrocardiographic waves (at the lower right side of (b)) are displayed at the same time. Furthermore, Fig. 4 is an enlarged view of a right half portion of Fig. 3 (b) . Fig. 3(b) and Fig. 4 show a B mode image and an M mode image containing the upper and lower walls of the blood vessel, an electrocardiogram, a cursor, etc. Particularly, the electrocardiographic waves are displayed at a substantially constant interval in synchronism with heartbeat of the examinee. Furthermore, Fig. 3(c) shows a display example in which the elastic image is superposed on the ultrasonic tomogram. In Fig. 3 (c), an elastic image display ROI is set in the ultrasonic tomogram of (b) diagram (at the left side of (b)) and the elastic image is determined in ROI and superposed on the ultrasonic tomogram. In order to make the following description simple, the R time point of the electrocardiogram (the position representing the R wave) is set as a reference point for the measurement although it is not particularly limited.

After the ultrasonic image as shown in Fig. 3(b) is displayed on the display means 9, a user freezes the ultrasonic image by using the freeze button 15a of the operation table 15. It is confirmed in the post-freeze ultrasonic image that a maximum expansion stage at which the blood vessel wall of the carotid artery expands at the maximum appears at the contraction stage of the cardiac ventricle, that is, after a fixed time difference from the R time point which is the pump-out timing of blood from the heart into the whole body (the time point of (1) of Fig. 4). This is because there exists a distance between the heart pumping out blood and the carotid artery as a target. Thereafter, the blood amount in the blood vessel decreases gradually, and thus the blood vessel enters the contraction stage. The place at which the blood vessel wall has completely contracted is the contraction end stage (the place of (2) of Fig. 4). The same phenomenon repeats at a cardiac cycle in an real-time ultrasonic wave image. Furthermore, the same phenomenon occurs in other arteries without limiting to the carotid artery.

In consideration of the foregoing description will be described a first example of this embodiment in which a period for which a moving tissue is rapidly displaced is selected and an elastic image is achieved by using the RF frame data (that is, data equivalent to a tomogram) of the period concerned, thereby acquiring a stable elastic image. For example, the period from the time point of the contraction end stage of the artery till the time point of the maximum expansion stage is selected, and elastic images are continuously acquired by using the RF frame data of that period. For example, as shown in Fig. 4, a stable elastic image is acquired in a period Y for which the blood vessel wall is rapidly displaced in one heartbeat. In order to satisfy this, an elastic image is calculated by using RF frame data which are as continuous (adjacent) as possible on the time axis during the period $\gamma$.

A first example of this embodiment will be described in detail by using an example of acquiring an elastic image of a blood vessel wall displaced due to pulsation of the heart as an example of the moving tissue. First, in Fig. 4, the R time point is set as a reference, and the time difference between the reference point R and each of the time point a of the contraction end stage just close to the reference point R and the time point b of the maximum expansion stage is measured (the reference point is not necessarily set to the R time point as described above). Subsequently, the time measuring means 16 outputs the data read-out signal to the saving means 10 so that the RF frame data are transferred from the saving means 10 to the elasticity calculating means 11 during the period between the time point a of the contraction end stage and the time point b of the maximum expansion stage which are measured from the detected R time point. The elasticity calculating means 11 executes the calculation of the elastic image by using the RF frame data of this period. The elastic image acquired by the calculation is displayed on the display means 9 through the color signal information exchange means 12 and the switching adding means 8. AS described above, elastic images are continuously acquired during the period for which the blood vessel wall is rapidly displaced.

Here, two methods of measuring a desired time point such as the time point a and the time point b of Fig. 4 will be described. As a first method of measuring a desired time point, a manual measurement method of the present invention and an example of the time measuring means 16 for enabling the above method will be described.

Fig. 5 shows an example of the construction of the time measuring means 16. The time measuring means 16 is equipped with a selector 16a for selecting a measuring mode, a manual measuring unit 16b, an automatic measuring unit 16c, and a data transfer signal output unit 16d. The manual measuring unit 16b and the automatic measuring unit 16c are connected to each other in parallel.

AT the manual measuring time, the manual measuring unit 16b is selected by the selector 16a in the time measuring means 16, and the time difference between the time point a and the R time point as a reference as shown in Fig. 4, and the time difference between the time point b and the R time point are measured. In general, the ultrasonic diagnostic apparatus has the function of measuring the distance between two points and measuring the time from an image after freeze by using a caliper (cursor), and this function is utilized in the measurements for various kinds of diagnosis. The same measuring method as described above is also used in the manual measuring unit 16b according to the present invention. A start point and an end point are determined by using the caliper after an image to be measured is determined, and the time between the start point and the end point is measured.

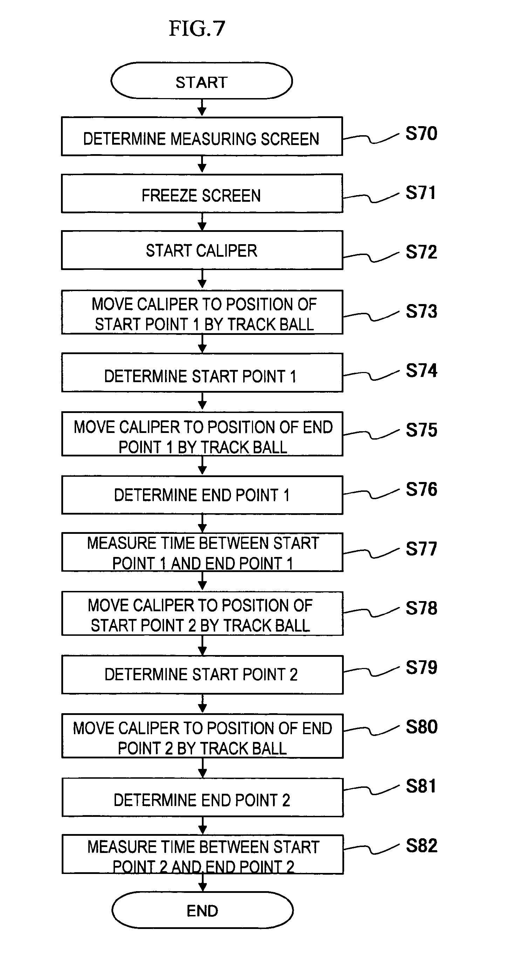

The measuring procedure of the manual measurement will be described with reference to Figs. 6 and 7. Fig. 6 is a diagram at the manual measurement time, and Fig. 7 is a flowchart. The acquisition of the elastic image on the basis of the selection of the acquisition period of the elastic image by using the manual measurement method is carried out in only the freeze mode. After an ultrasonic image to be measured is determined (S70), the ultrasonic image is frozen by the freeze button 15a of the operation table 15 (S71). At this time, the caliper is started by the caliper start button 15b of the operation table 15 (S72). The start point 1 of the caliper is moved to the R time point of the electrocardiogram by using the track ball 15c (S73), and the start point 1 is determined by the enter button 15d of the operation table 15 (S74). Thereafter, the caliper is moved to a place at which the interval of the blood vessel wall is most contracted (contraction end stage) (S75), and the end point 1 is determined (t1 of Fig. 6, S76). The time t1 between the determined start point 1 and end point 1 is measured (S77). Furthermore, the caliper is moved to the R time point of the electrocardiogram, that is, the position of the start point 2 by the track ball (S78), and the start point 2 is determined to the R time point of the electrocardiogram (S79). The caliper is moved to a place which the blood vessel wall is most expanded (maximum expansion stage) (S80), and the end point 2 is determined (t2 of Fig. 6, S81). The time t2 between the determined start point 2 and end point t2 is measured (S82).

Through the above operation, the time differences t1, t2 at the two places of the contraction end stage and maximum expansion stage of the blood vessel wall with respect to the R time point of the electrocardiogram are measured. The measured time differences t1, t2 are displayed at the lower portion of the screen (t1 = Xsec, t2 = Ysec).

The measuring timing is not limited to the above t1, t2. That is, the contraction end stage and the maximum expansion stage may be set to t2,t1. Furthermore, another measuring timing may be set between the contraction end stage and the maximum expansion stage.

Next, an automatic measuring method of the present invention as a second method for measuring a desired time point and an example of the time measuring means 16 for implementing this method will be described. Acquisition of an elastic image based on the selection of the elastic image acquiring period by using the following automatic measuring method may be performed in each of the real-time mode and the freeze mode, and the measuring method in the freeze mode will be described below.

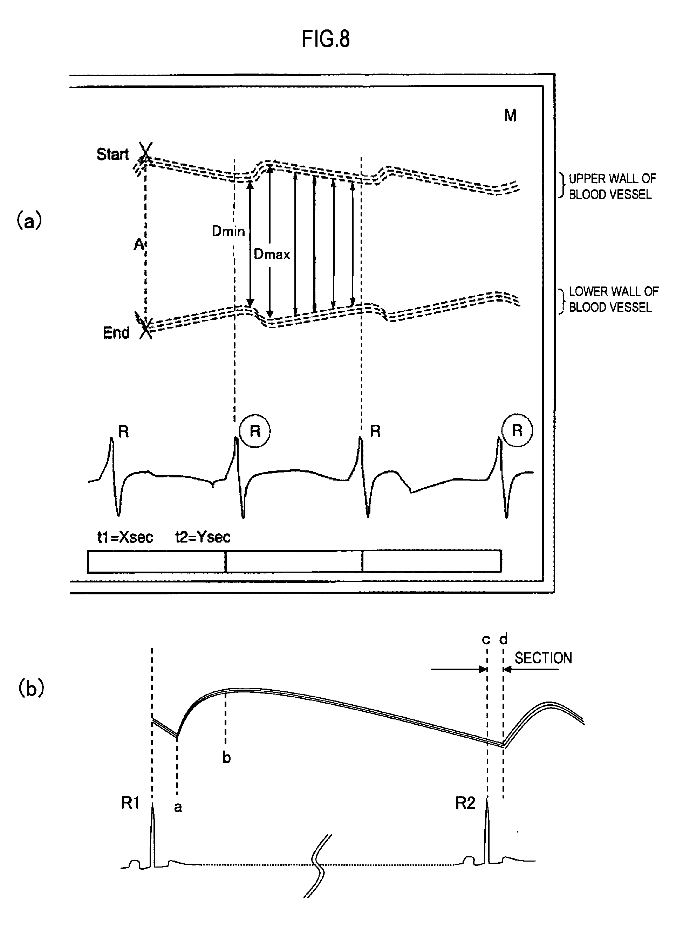

Fig. 8 shows the automatic measuring method. According to the automatic measuring method, the distance D inside the blood vessel with the R time point of the electrocardiogram set as a reference is automatically measured at a fixed interval, and the time differences at the places corresponding to the minimum distance Dmin and the maximum distance Dmax are automatically extracted. As in the case of the manual measurement, the user selects the automatic measuring unit 16c by the selector 16a, and a screen to be measured is determined, a measuring range A as shown in Fig. 8 is set by the caliper starting button 15b of the operation table 15. After the measuring range A is set, the mode is shifted to an automatically measuring mode of several seconds by the automatically measuring button 15e of the operation table 15. In this automatically measuring mode, some R time point is set as a reference, and then the distance between the upper and lower walls of the blood vessel between the reference R time point and the subsequent R time point is measured. The measurement is carried out every in-circle R (a symbol of R surrounded by a circle in Fig. 8) which is located at every other R time point as in the case of the manual measurement although it is not particularly limited.

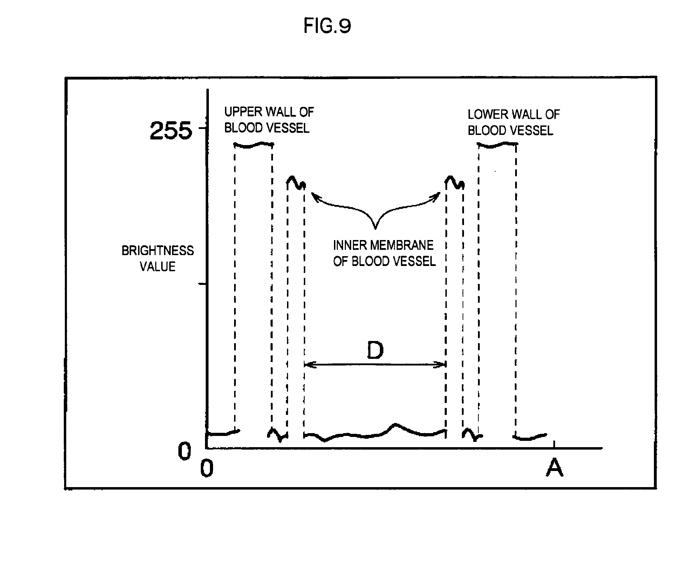

An example of the automatic measurement of the distance D in the blood vessel will be described with reference to Figs. 9 to 11.

As a method of measuring the distance D in the blood vessel , the distance D may be measured by using the difference in brightness value in the measuring range A, for example. Fig. 9 shows a brightness value distribution in the depth direction from the Start time point to the End time point in the measuring range A of Fig. 8. The ordinate axis of the brightness value distribution represents the brightness value, and the abscissa axis represents the distance in the depth direction from the Start time point to the End time point. In Fig. 9, it is generally known that the upper and lower walls and inner membrane of the blood vessel provide high brightness. The brightness value of these portions is near to 255. An area having an extremely low brightness value exists between the upper and lower walls and the inner membrane.

The differential value of the brightness value is calculated according to the following equation from the Start time point to the End time point in the depth direction.

$$\mathrm{Pix\_sub} = \left| \mathrm{Pix}(n) - \mathrm{Pix}(n+1) \right| \quad \dots \ (2)$$

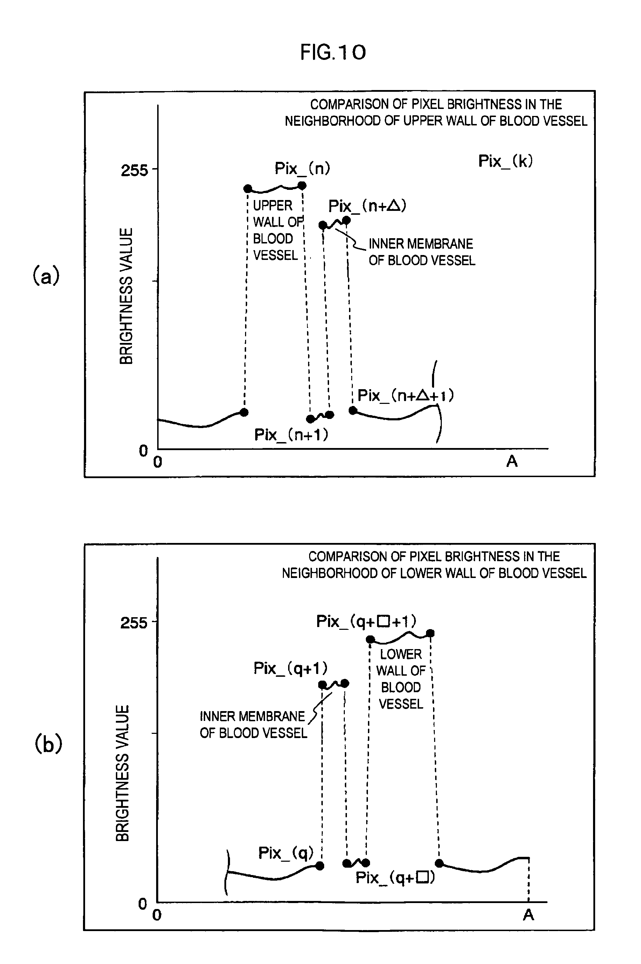

(n represents an integer) Two places at which the brightness value or the difference value between the upper wall and the inner membrane is extremely large appear in the neighborhood of the upper wall of the blood vessel. This will be described with reference to Fig. 10 and the following equations (3) and (4). Fig. 10(a) is an enlarged view of the neighborhood of the upper wall of the blood vessel in Fig. 9. When the pixel values of a point of interest (a place where the brightness value is extremely large) are represented by Pix (n), Pix (n+1), Pix (n+$\Delta$), Pix (n+$\Delta$+1) ($\Delta$ represents a natural number) and the threshold value is represented by $\beta$, the difference value of the brightness value is represented by the following equations. In the following equations, the place (n+$\Delta$+1) corresponding to (3), (4) is set as a start point. The threshold value P is not uniquely determined, but freely changeable.

$$\mathrm{Pix\_sub1} = \left| \mathrm{Pix}(n) - \mathrm{Pix}(n+1) \right| \ \rightarrow \ (\mathrm{Pix\_sub1} > \beta) \quad \dots \ (3)$$

$$\texttt{Pix\_sub2=}\left|\texttt{Pix(n+}\Delta\texttt{)-Pix(n+}\Delta\texttt{+1)}\right| \ \rightarrow \ \texttt{(Pix\_sub2>}\beta\texttt{)} \quad \ldots \texttt{(4)}$$

Two places at which the brightness value or the difference value between the inner membrane and the lower wall is extremely large also appear in the lower wall of the blood vessel. This will be likewise described by using Fig. 10 (b) and the following equations (5), (6). The pixel values at a point of interest (a place where the brightness value is extremely large) are represented by Pix (q), Pix (q+1), Pix (q+), Pix(q+ +1) (represents a natural number) and the threshold value is represented by β, and the difference value of the brightness value is calculated as follows. The place (q) shown in the following equations (5), (6) is set as an end point. The threshold value β is not uniquely determined, but freely changeable.

$$\texttt{Pix\_sub3=}\left|\texttt{Pix(q)-Pix(q+1)}\right| \ \rightarrow \ \texttt{(Pix\_sub3>}\beta\texttt{)} \quad \ldots \texttt{(5)}$$

$$\texttt{Pix\_sub4=}\left|\texttt{Pix(q+ )-Pix(q+ +1)}\right| \ \rightarrow \ \texttt{(Pix\_sub4>}\beta\texttt{)} \quad \ldots \texttt{(6)}$$

Through the above operation, the start point and the end point are determined. The above calculation in the depth direction is repeated while shifting the calculation line in the time direction (lateral direction), thereby measuring the distances Dmax and Dmin of Fig. 8(a).
The start point and the end point of the calculation line can be defined as follows. For example, the motion of the blood vessel wall from the time point of R1 as one R wave shown in Fig. 8(b) till the time point of R2 as the next R wave is considered in detail. The contraction end stage of the blood vessel comes after the period (c-d) from R2, and the pulsation substantially falls into the contraction end stage from c point to d point, and thus no remarkable difference appears between the blood vessel walls in this section. Therefore, the R2 point can be set to the end point of the automatic measurement. The distance between the blood vessel walls is first reduced at d=a point. Therefore, the a point is set to the start point, and the period from the a point to the end point R2 can be set to a period effective to the measurement.
Finally, the R time point is set as a reference, and the time difference t1, t2 between the R time point and the time point at which the distance D between the blood vessel walls is equal to Dmin,Dmax is calculated, whereby the automatic measurement can be performed.
As described above, the automatic measurement is carried out in the freeze mode, and elastic images can be continuously acquired for the period between the time points at which the distance D between the blood vessel walls is equal to Dmin and Dmax.
On the other hand, in the real-time mode, the processing of the automatic measuring method described above is repeated for the adj acent R-R period. That is, every adj acent R-R period, the time points at which the distance D between the blood vessel walls is equal to Dmin, Dmax are detected and the time differences t1, t2 from the reference time point (normally, it is set to the first R time point) for the adjacent R-R period are calculated. Furthermore, elastic images continuous for the period between the time points at which the distance D between the blood vessel walls is equal to Dmin, Dmax are repetitively acquired every adjacent R-R period.
It is possible to acquire singular time points in one heartbeat by any one of the manual measuring method and the automatic measuring method. An elastic image is acquired by using RF frame data having a large displacement amount between these singular time points, whereby an elastic image having high image quality can be stably acquired.
Next, an example of stably acquiring an elastic image having high image quality by controlling the extraction frequency of the elastic image in accordance with the displacement amount of the moving tissue will be described as a second example of this embodiment.
For example, the extraction frequency of the elastic image is increased/reduced in accordance with the displacement amount of the moving tissue. Alternatively, a first period for which the displacement of the moving tissue is steep and a second period for which the displacement of the moving tissue is more moderate than that of the first period are selected, and the acquiring frequency of the elastic image in the first period is set to a larger value than the second period.
This example can be performed in each of the real-time mode and the freeze mode, and the example in the freeze mode will be described hereunder.
In the second example, the automatic measuring method described in the first example is put into practical use, and the second embodiment will be described with reference to Fig. 8. First, as in the case of the automatic measuring method described in the first example, the user selects the automatic measuring unit 16c by the selector 16a of the time measuring means 16, and a screen to be measured is determined. Thereafter, a measuring range A as shown in Fig.

8 is set by the caliper start button 15b of the operation table 15. After the measuring range A is set, the mode is shifted to the automatic measuring mode of several seconds by the automatic measuring button 15e of the operation table 15. The automatic measuring method of the distance D in the blood vessel which is carried out in the automatic measuring mode is the same as the automatic measuring method described in the first example, and thus the description thereof is omitted.

The automatic measuring unit 16c calculates the differential value of the distance D in the blood vessel which is calculated every calculation line, and the differential value is set as the displacement amount of the blood vessel wall between adjacent lines. For example, the differential value $\Delta(N, N+1)$ between the distance $D(N)$ in the blood vessel of a reference calculation line N and the distance $D(N+1)$ in the blood vessel of a comparative calculation line (N+1) is calculated. Here, the blood vessel wall is sharply displaced for the period a-b shown in Fig. 8(b), and thus this period has a large displacement amount of the blood vessel wall. On the other hand, the blood vessel wall is moderately displaced for the period b-c shown in Fig. 8(b), and thus this period has a small displacement of the blood vessel wall.

Therefore, if the calculated difference $\Delta(N, N+1)$ is equal to a predetermined threshold value K or more, the period is regarded as the period a-b (first period), and the data transfer signal output unit 16d outputs a data read-out signal to the saving means 10 so that the RF frame data corresponding to the time points of the two calculation lines N, N+1 are transferred from the saving means 10 to the elasticity calculating means 11. The elasticity calculating means 11 creates an elastic image by using these two RF frame data.

On the other hand, if the calculated difference $\Delta(N, N+1)$ is less than the predetermined threshold value K, the period is regarded as the period b-d (second period), and the difference $\Delta(N, N+2)$ between the distance $D(N+2)$ in the blood vessel of the next comparative calculation line N+2 and the distance $D(N)$ in the blood vessel of the reference calculation line N is calculated. This processing is repeated while shifting the comparative calculation line until the calculated differential value $\Delta$ is equal to the predetermined threshold value K or more. An elastic image is created every time the differential value $\Delta$ is equal to the predetermined threshold value K or more. When the elastic image is created, the data transfer signal output unit 16d outputs the data read-out signal to the saving means 10 so that the RF frame data corresponding to the time points of the two calculation line are transferred from the saving means 10 to the elasticity calculating means 11 as described above, and the elasticity calculating means 11 creates the elastic image by using these two RF frame data.

After the elastic image is created, the same calculation is repeated on the next adjacent two calculation lines with these calculation lines set as a reference calculation line and a comparative calculation line. Furthermore, when the next R time point is detected, the same calculation is likewise repeated on the adjacent two calculation lines with the calculation lines set as a reference calculation line and a comparative calculation line.

However, the displacement amount of the blood vessel wall is small for the period c-d from the R time point to the contraction end stage shown in Fig. 8(b), and thus it is possible to prohibit creation of an elastic image in accordance with the threshold value K. Alternatively, the calculated distance D in the blood vessel is reduced for the period c-d, and thus calculated strain may be removed to prohibit creation of the elastic image through the threshold value processing using a strain average value by the elasticity analyzer 11c described above.

In the foregoing description, the displacement amount of the blood vessel wall is compared with the threshold value K to judge whether the period is the period a-b or the period b-d, and the frequency of acquiring elastic images is controlled. The elastic image acquisition may be controlled by merely comparing the displacement amount and the threshold value K without the judgment as to the period, whereby the control of the elastic image acquiring frequency in accordance with the displacement amount can be performed in a stepless style. Furthermore, the foregoing description relates to the example of controlling the acquisition of the elastic image in the freeze mode, however, the acquisition frequency of the elastic image can be controlled on real-time basis by executing the automatic measurement of the distance D in the blood vessel in the real-time mode and the comparison between the displacement amount of the blood vessel wall and the threshold value K on real-time basis and properly renewing the period of acquiring the elastic image.

By the processing as described above, elastic images can be acquired highly frequently during the period for which the blood vessel wall is sharply displaced because the displacement amount is large in this period, and elastic images can be acquired by using RF frame data having a sufficient displacement amount by reducing the elastic image acquiring frequency during the period for which the blood vessel wall is moderately displaced. That is, the elastic image having high image quality can be acquired stably by controlling the elastic image acquiring frequency in accordance with the displacement amount of the blood vessel wall.

As described above, according to this embodiment, the elastic image having high image quality can be stably acquired by acquiring one set of RF frame data containing a proper displacement amount and measuring the displacement amount of the blood vessel wall in the period for which the blood vessel wall is sharply displaced as seen from the contraction end stage of the blood vessel wall to the maximum contraction period in one heartbeat, or in accordance with the displacement value of the blood vessel.

(Second Embodiment)

Next, a second embodiment of the present invention will be described. In this embodiment, the selection of the elastic image acquiring period and the control of the acquiring frequency are performed in accordance with the displacement amount of the tissue in the thoracoabdominal site which is caused by breathing motion. The displacement amount of the tissue in the thoracoabdominal site is detected by using tomogram image (B mode image) data, for example. The different point from the first embodiment resides in the displacement amount detecting method based on the difference in the type of the body motion of the examinee and the specific elastic image acquisition control, and the other matters are the same. Only the different portion will be described hereunder, and the description of the same portion is omitted. This embodiment can be also implemented in any one of the real-time mode and the freeze mode.

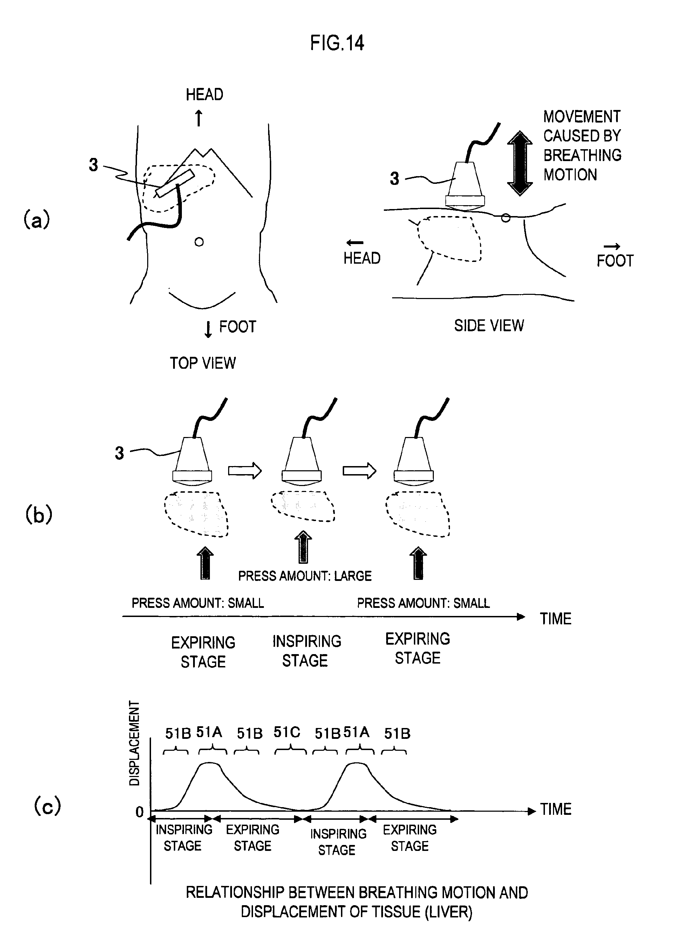

An example of acquiring an elastic image of a liver as an example of this embodiment will be described with reference to Fig. 14. Fig. 14 (a) show a top view (left diagram) showing the state that the probe 3 is brought into contact with the abdomen of an examinee to acquire a tomogram of the liver, and a side view (right diagram) when viewed from the left side of the examinee. Fig. 14(b) shows an example of an aspect that the press to the liver is periodically varied due to breathing.

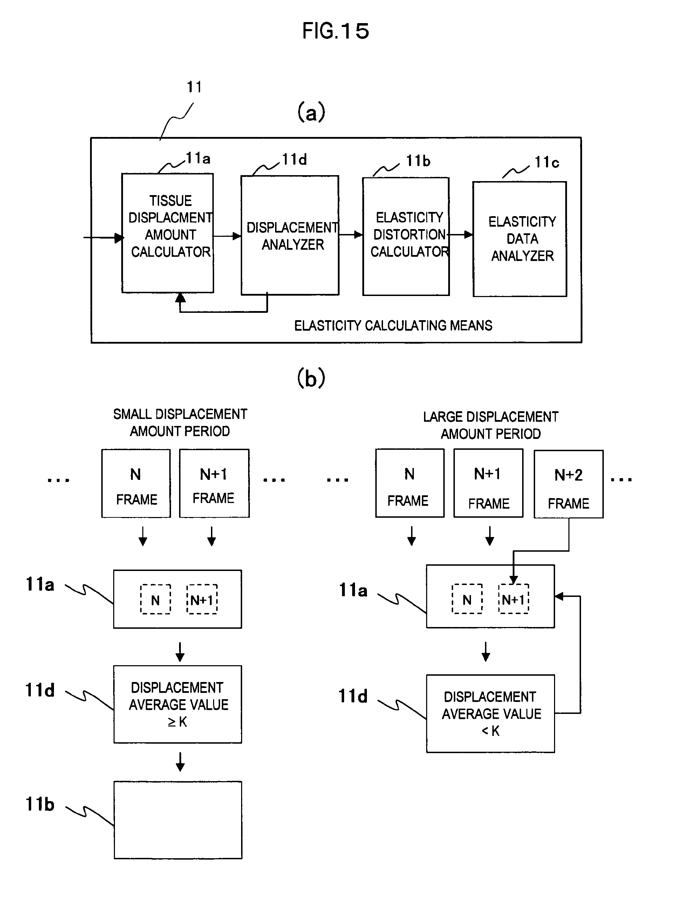

The press amount to the liver is periodically varied in accordance with the periodical vertical movement of the diaphragm which is caused by breathing. That is, as shown at the left diagram of Fig. 15 (b), the diaphragm moves to the head side in a breath-out stage (an expiring stage) and thus the press amount to the liver is reduced. As a result, the displacement amount (strain amount) of the liver becomes small and thus the liver expands greatly. Thereafter, when the breath-out stage is shifted to a breath-in stage (inspiration period), the diaphragm moves to the foot side and thus the press amount to the liver is increased. As a result, the displacement amount (strain amount) to the liver is increased and thus the liver is contracted to a small size. Thereafter, the breath-in stage is shifted to the breath-out stage, and the displacement amount (strain amount) of the liver is reduced. As described above, the press amount to the liver is periodically varied in accordance with breathing, and the displacement amount (strain amount) of the liver is also periodically varied in accordance with the periodical variation of the press amount.

Fig. 14 (C) shows the aspect of the periodical variation of the displacement amount (strain amount) of the liver. In general, the breathing motion has a breathing ceasing period in some period from the end of breath-out till the start of breath-in, and a rapid breathing period (inspiring stage) exists after the ceasing period. Then, breathing instantaneously stops in the neighborhood of the time point at which the inspiring stage turns to the expiring stage. Thereafter, the breath-out period is started. The displacement amount of the liver and the time-variation (the inclination of the graph) of the displacement amount also vary while following the breathing motion as described above. Specifically, as shown in Fig. 14 (c), in the shift period (51A) from the inspiring stage to the expiring stage and the shift period (51C) from the expiring stage to the inspiring stage, the breathing motion is moderate and thus the displacement amount of the liver is also small. On the other hand, the periods before and after the shift period are the period (51B) for which the displacement amount of the liver is large because the breathing motion is sharp.

A method of detecting the displacement amount of the inner tissue due to the breathing motion and the displacement direction will be described with reference to Fig. 15.
Fig. 15(a) shows an example of the construction of the elasticity calculating means 11 suitable for implementing this example, and a displacement analyzer 11d is further inserted between the tissue displacement amount calculator 11a and the elasticity/strain calculating unit 11b in the construction shown in Fig. 11. The displacement amount calculated by using plural RF frame data in the tissue displacement amount calculator 11a is input to the displacement analyzer 11d, and the displacement amount concerned is compared with a predetermined threshold value in the displacement analyzer 11d. When the displacement amount is equal to a threshold value or more, the displacement amount distribution at that time is output to the elasticity/strain calculator 11b, and an elasticity image is calculated by using the displacement amount distribution in the elasticity/strain calculator 11b.

Fig. 15(b) shows the summary of processing flow of detecting the period for which the displacement amount calculated in the elasticity calculating means 11 shown in Fig. 15(a) is large. The R time point is set as a reference, and RF frame data of two frames in time-sequentially generated RF frame data ..., N, N+1, N+2, N+3, N+4, ... are input to the tissue displacement amount calculator 11a in the elasticity calculating means 11 at all times. The tissue displacement amount calculator 11a calculates the displacement amount and the displacement direction (that is, displacement vector) at each point by using the two input RF frame data. The displacement analyzer 11d analyzes the displacement amount calculated in the tissue displacement amount calculator 11a. For example, the average value of the calculated displacement amount, etc. are calculated.

As a result, in the shift period from the inspiring stage to the expiring stage in the period 51A of Fig. 14(c) and the period from the expiring stage to the inspiring stage in the period 51C, the breathing motion is moderate and thus the calculated displacement amount (average amount) is small.
Conversely, in the inspiring stage or expiring stage in the period 51B, the breathing motion is sharp, and thus the calculated displacement amount (average value) is large. Furthermore, which time point or period can be recognized from the displacement direction. For example, in the case of the displacement to the head side, the expiring stage is recognized, and in the case of the displacement to the foot side, the inspiring stage is recognized. If the displacement direction varies from the head side to the foot side, the shift from the expiring stage to the inspiring stage and also that time point can be recognized. If the displacement direction varies from the foot side to the head side, the shift from the inspiring stage to the expiring stage and also that time point can be recognized.

By using the information on the displacement amount and the displacement direction thus detected, the continuous acquisition of elastic images of the tissue in the thoracoabdominal site and the control of the extraction frequency of the elastic images in accordance with the displacement amount can be performed as in the first embodiment described above. These operations will be specifically described.

First, a first example of this embodiment of continuously acquiring elastic images of the tissue in the thoracoabdominal site will be described. The sharp period of the displacement amount at the expiring stage and the inspiring stage recognized as described above is selected, and RF frame data are continuously acquired during that period. The acquisition of the RF frame data is the same as the first embodiment.

The selection of the period of the sharp displacement can be performed as follows, for example. The displacement analyzer 11d compares the displacement amount (average value) calculated in the displacement analyzer 11d during the expiring stage with a predetermined threshold value L. A period for which the displacement amount (average value) continues to be equal to the threshold value L or more from the time point when the displacement amount (average value) increases to be larger than the threshold value L till the time point when the displacement amount decreases to be smaller than the threshold value L is selected. The displacement amount distribution is output to the elasticity/strain calculator within this period, thereby continuously acquiring elastic images. Likewise, during the inspiring stage, a period for which the displacement amount (average value) continues to be equal to the threshold value L or more is selected, and elastic images are continuously acquired during this period. The threshold value L may be set to different values between the inspiring stage and the expiring stage.

As described above, if the period for which the displacement amount (average amount) continues to be equal to the threshold value L or more is selected, the displacement amount of the tissue is large in these periods, and thus the displacement amount can be calculated stably and accurately. Accordingly, the elastic images having high image quality can be stably acquired during these periods.

Next, a second example of this embodiment for controlling the elastic image acquiring frequency in accordance with the displacement amount of the tissue in the thoracoabdominal site will be described. By using the information of the displacement amount recognized as described above, elastic images are acquired at high frequency, preferably at a frame rate of tomograms during the period (51B) for which the displacement amount is increased. On the other hand, elastic images are acquired at a low frequency, in extreme case, no elastic image is acquired during the periods (51A and 51C) for which the displacement amount is reduced. As described above, elastic images are acquired at high frequency during the period for which the displacement amount is large, whereby elastic images having high image quality can be stably achieved.

Specifically, the elastic image acquiring frequency can be controlled in accordance with the displacement amount by controlling the elastic image acquisition through the comparison between the displacement amount and a predetermined threshold value. That is, if the calculated displacement amount (average value) is equal to a predetermined threshold value M or more, the period is regarded as the period 51B (first period), and the displacement amount distribution calculated in the tissue displacement amount calculator 11a is output to the elasticity/strain calculator 11b to acquire elastic images. On the other hand, if the calculated displacement amount (average value) is less than the threshold value M, the period is regarded as the period 51A or 51C (second period), and the displacement amount distribution calculated in the tissue displacement amount calculator 11a is not output to the elasticity/strain calculator 11b. Accordingly, no elastic image is acquired during the periods 51A and 51C.

In the real-time mode, the comparison between the displacement amount (average value) and the threshold value M is carried out on real-time basis, and the elastic image acquiring period is properly renewed.

When the calculated displacement amount (average value) is less than the threshold value M, the (N+1)-th RF frame data are deleted from the memory space of the tissue displacement amount calculator 11a, and in place of that data, (N+2)-th RF frame data are input. The displacement amount (average value) calculation is carried out again between the RF frame data of the (N)-th frame and the (N+2)-th frame, and the calculation result is compared with the threshold value M to judge whether elastic images are acquired or not. This processing is repeated until the calculated displacement amount (average value) is equal to the predetermined threshold value M or more. An elastic image is created every time the displacement amount (average value) is equal to the predetermined threshold value M or more. In addition, new two adjacent RF frame data are input to the memory space of the tissue displacement amount calculator 11a, and the above calculation is repeated.

In the periods 51A and 51C, the displacement amount (average value) of the tissue in the thoracoabdominal site is reduced and the calculated strain value (average value) is also reduced. Therefore, as in the case of the first embodiment described above, it is possible to prohibit creation of the elastic image in accordance with the threshold value M. Or, the calculated strain may be removed to prohibit creation of the elastic image by the threshold value processing using the strain average value of the elasticity analyzer 11c described above.

Furthermore, in the foregoing description, the displacement amount is compared with the threshold value M to judge the period 51B or the period 51A or 51C, and the elastic image acquiring frequency is controlled. As in the case of the first embodiment, the elastic image acquisition may be controlled by merely comparing the displacement amount and the threshold value M without judging the period, whereby the control of the elastic image acquiring frequency in accordance with the displacement amount can be performed in a stepless style.

In the period for which the tissue displacement is sharp and the displacement amount becomes large between the adj acent RF frame data, the elastic images can be acquired at high frequency by executing the processing as described above. On the other hand, in the period for which the tissue displacement is moderate and the displacement amount becomes small between the adjacent RF frame data, elastic images can be acquired by using RF frame data having a sufficient displacement amount by reducing the elastic image acquiring frequency. That is, the elastic images of high image quality can be stably acquired by controlling the elastic image extracting frequency in accordance with the displacement amount of the tissue in the thoracoabdominal site.

In the foregoing description, the displacement amount of the tissue is detected by using time-sequentially generated RF frame data, and the elastic images are generated during the period for which the displacement amount is large. The breathing motion may be monitored by using external detecting means for detecting the breathing motion without using the RF frame data and the elastic image acquisition may be controlled in accordance with the displacement amount of the breathing motion.

As described above, according to this embodiment, even when the displacement of the tissue displacement amount is varied due to breathing, the elastic images of high image quality can be stably acquired.

The embodiments of the ultrasonic diagnostic apparatus and the ultrasonic elastic image acquiring method according to the present invention have been described above. However, the present invention is not limited to the contents disclosed in the description of the above embodiments, and other embodiments may be achieved in consideration of the subject matter of the present invention. For example, in the above embodiments, the blood vessel and the tissue in the thoracoabdominal site are targets. However, any biomedical tissue may be used insofar as the displacement occurs in accordance with pulsation or breathing motion. Furthermore, the R-wave of the electrocardiogram is used to set the reference time point, however, the present invention is not limited to this mode. For example, the motion of a living body such as breathing or the like (for example, motion of diaphragm) may be detected and used (for example, the probe 3 is provided with a position sensor and the motion is detected by using the position sensor). Furthermore, the motion of a living body when the living body is substantially periodically displaced by action from the outside of an examinee such as a vibrator or the like may be detected and used.

In the description of the above embodiments, the elastic acquisition calculation is executed mainly after freeze. However, if the automatic detection method of the specific time point and the automatic detection method of the displacement amount are used, the elastic image extraction frequency control can be performed continuously or in accordance with the displacement amount even in a real-time image pickup operation.

Brief Description of the Drawings

[Fig. 1] is a block diagram showing a first embodiment of an ultrasonic diagnostic apparatus according to the present invention.
[Fig. 2] is generally detected electrocardiographic waves.
[Fig. 3] is a diagram showing a display example in which B mode/M mode/electrocardiographic waves are displayed.
[Fig. 4] is an enlarged view of M mode/electrocardiographic waves of Fig. 3.
[Fig. 5] is a block diagram showing time measuring means.
[Fig. 6] is a diagram showing manual measurement.
[Fig. 7] is a flowchart of the manual measurement.
[Fig. 8] is a diagram showing an automatic measuring method.
[Fig. 9] is a distribution diagram of brightness values in a measuring range A.
[Fig. 10] is a diagram showing the measurement of the distance D, wherein (a) is a diagram showing the comparison of pixel brightness in the neighborhood of the upper wall of the blood vessel, and (b) is a diagram showing the comparison of pixel brightness in the neighborhood of the lower wall of the blood vessel.
[Fig. 11] is a block diagram showing elasticity calculating means.
[Fig. 12] is a diagram showing an example of an operation table.
[Fig. 13] is a block diagram showing saving means.
[Fig. 14] is a diagram showing the press of a tissue in a thoracoabdominal site and the displacement amount due to breathing motion.

[Fig. 15] is a diagram showing an example of the construction of controlling an elastic image extracting frequency in accordance with the displacement amount and the processing flow.

Description of Reference Numerals

| | |
|---|---|
| 1 | transmitting means |
| 2 | transmitting/receiving separation means |
| 3 | probe |
| 4 | receiving means |
| 5 | phasing addition means |
| 6 | monochromatic signal processing means |
| 7 | monochromatic signal information converting means |
| 8 | switching addition means |
| 9 | display means |
| 10 | saving means |
| 11 | elasticity calculating means |
| 12 | color signal information exchanging means |
| 13 | ultrasonic control means |
| 14 | ultrasonic system control means |
| 15 | operation table |
| 16 | time measuring means |
| 17 | biomedical signal analyzing means |
| 18 | electrode clip |
| 19 | biomedical motion detecting means |
| 20 | supply means |

**Claims**

1. A ultrasonic diagnostic apparatus including an ultrasonic probe for transmitting/receiving ultrasonic waves to/from a site containing a moving tissue of an examinee; ultrasonic wave transmitting means for outputting an ultrasonic wave signal for driving the ultrasonic probe; morphological image acquiring means for acquiring a morphological image representing morphological information of the site containing the moving tissue from a reflected echo signal received by the ultrasonic probe; and elastic image acquiring means for acquiring an elastic image representing elastic information of the site containing the moving tissue by using a plurality of morphological image data, further comprising:

   displacement amount detecting means for detecting a displacement amount of the moving tissue by using the morphological image data; and
   selecting means for selecting an elastic image acquiring period on the basis of the displacement amount, wherein the elastic image acquiring means acquires an elastic image during the selected period.

2. The ultrasonic diagnostic apparatus according to claim 1, wherein the selecting means selects a period for which the displacement amount of the moving tissue is sharper than that of other periods.

3. The ultrasonic diagnostic apparatus according to claim 2, wherein the moving tissue is a desired blood vessel wall, and the selecting means selects a period from a time point when the desired blood vessel wall starts sharp displacement till a time point when the interval of the blood vessel walls is maximum.

4. The ultrasonic diagnostic apparatus according to claim 2, wherein the moving tissue is a tissue in a thoracoabdominal site, and the selecting means selects a period which is within an expiring stage or inspiring stage of breathing motion and in which the displacement amount of the tissue in the thoracoabdominal site is equal to a predetermined threshold value or more.

5. The ultrasonic diagnostic apparatus according to any one of claims 2 to 4, wherein the elastic image acquiring means continuously acquires the elastic images during the selected period.

6.  The ultrasonic diagnostic apparatus according to claim 1, wherein the elastic image acquiring means controls an elastic image acquiring frequency in accordance with the displacement amount of the moving tissue.

7.  The ultrasonic diagnostic apparatus according to claim 6, wherein the elastic image acquiring means controls the elastic image acquiring frequency so that the elastic image acquiring frequency is increased or reduced in accordance with the displacement amount of the moving tissue.

8.  The ultrasonic diagnostic apparatus according to claim 6, wherein the selecting means selects a first period for which the displacement of the moving tissue is sharp and a second period for which the displacement of the moving tissue is more moderate than that of the first period, and the elastic image acquiring means increases the elastic image acquiring frequency in the first period to be larger than that of the second period.

9.  The ultrasonic diagnostic apparatus according to claim 8, wherein the moving tissue is a desired blood vessel wall, the first period is from a time point when the desired blood vessel wall starts sharp displacement till a time point when the interval of the blood vessel wall is maximum, and the second period is from a time point when the interval of the blood vessel wall is maximum till a time point when the blood vessel wall starts sharp displacement.

10. The ultrasonic diagnostic apparatus according to claim 8, wherein the moving tissue is a tissue in a thoracoabdominal site, the first period is a period between a time point when the inspiring stage is changed to the expiring stage and a time point when the expiring stage is changed to the inspiring stage, and the second period is a period containing a time point when the breathing motion is changed from the inspiring stage to the expiring stage or a period containing a time point when the breathing motion is changed from the expiring stage to the inspiring stage.

11. The ultrasonic diagnostic apparatus according to claim 3 or 9, wherein the morphological image acquiring means acquires an M mode image as the morphological image, and the displacement amount detecting means detects the displacement amount of the desired blood vessel wall by using the M mode image.

12. The ultrasonic diagnostic apparatus according to claim 11, further comprising input means for inputting information indicating on the M mode image a time point when the desired blood vessel wall starts sharp displacement and a time point when the interval of the blood vessel wall is maximum.

13. The ultrasonic diagnostic apparatus according to claim 11, wherein the displacement amount detecting means detects the interval of the desired blood vessel wall by using variation of the brightness value of the M mode.

14. The ultrasonic diagnostic apparatus according to claim 4 or 10, wherein the morphological image acquiring means acquires a tomogram as the morphological image, and the displacement amount detecting means detects the displacement amount of the tissue in the thoracoabdominal site by using plural tomogram data.

15. The ultrasonic diagnostic apparatus according to claim 1, wherein the displacement amount detecting means detects the displacement amount on real-time basis in accordance with the morphological image data acquired on real-time basis, the selecting means renews the selecting period in accordance with the displacement amount detected on real-time basis, and the elastic image acquiring means acquires the elastic image every renewed period.

16. The ultrasonic diagnostic apparatus according to claim 1, wherein the selecting means selects the acquiring period in accordance with frozen morphological image data.

17. The ultrasonic diagnostic apparatus according to claim 1, further comprising saving means for saving a plurality of morphological image data, wherein the elastic image acquiring means acquires the morphological image data corresponding to the period selected by the selecting means from the saving means to generate the elastic image.

18. A method of acquiring an ultrasonic elastic image of a site containing a moving tissue of an examinee, comprising:

    (a) a step of acquiring a morphological image representing morphological information of the site containing the moving tissue;
    (b) a step of acquiring an elastic image representing elastic information of the site containing the moving tissue by using a plurality of morphological image data; and

(c) a step of repeating the steps (a) and (b), wherein in the elastic image acquiring step, the displacement amount of the moving tissue is detected by using the morphological image data, and an acquiring period of the elastic image is selected on the basis of the displacement amount.

**19.** The ultrasonic elastic image acquiring method according to claim 18, wherein an M mode image containing a desired blood vessel wall is acquired as the morphological image in the morphological image acquiring step, and in the elastic image acquiring step, a period for which the displacement amount of the blood vessel wall is more sharper than that of other periods is selected by using the data of the M mode image, and elastic images are continuously acquired during the period concerned.

**20.** The ultrasonic elastic image acquiring method according to claim 18, wherein in the morphological image acquiring step, a tomogram of the site containing the moving tissue is acquired as the morphological image, and in the elastic image acquiring step, a period for which the displacement amount of the moving tissue is sharper than that of other periods is selected by using the data of the tomogram , and elastic images are continuously acquired during the period concerned.

FIG.1

## FIG. 2

P-WAVE : CONTRACTION STAGE OF ATRIUM
QRS WAVE: CONTRACTION STAGE OF CARDIAC VENTRICLE
T WAVE : STAGE AT WHICH HEART RETURNS TO ORIGINAL

**FIG. 3**

(a)

(b)

(c)

# FIG. 4

UPPER WALL OF BLOOD VESSEL

LOWER WALL OF BLOOD VESSEL

Freeze

**16**  FIG. 5

TIME MEASURING
MEANS
**16d**

**16a**

**16b**

MANUAL
MEASURING UNIT

SELECTOR

DATA TRANSFER
SIGNAL OUTPUT
UNIT

AUTOMATIC
MEASURING UNIT

**16c**

FIG. 6

START POINT 1    ENDPOINT 1
START POINT 2    END POINT 2

t1

t2

R    R    R    R

t1=Xsec    t2=Ysec    Freeze

# FIG.7

```
          ┌─────────────┐
          │    START    │
          └──────┬──────┘
                 ↓
   ┌──────────────────────────────┐
   │  DETERMINE MEASURING SCREEN  │─── S70
   └──────────────┬───────────────┘
                  ↓
   ┌──────────────────────────────┐
   │         FREEZE SCREEN        │─── S71
   └──────────────┬───────────────┘
                  ↓
   ┌──────────────────────────────┐
   │         START CALIPER        │─── S72
   └──────────────┬───────────────┘
                  ↓
   ┌──────────────────────────────┐
   │    MOVE CALIPER TO POSITION OF│─── S73
   │   START POINT 1 BY TRACK BALL │
   └──────────────┬───────────────┘
                  ↓
   ┌──────────────────────────────┐
   │     DETERMINE START POINT 1  │─── S74
   └──────────────┬───────────────┘
                  ↓
   ┌──────────────────────────────┐
   │   MOVE CALIPER TO POSITION OF │─── S75
   │    END POINT 1 BY TRACK BALL  │
   └──────────────┬───────────────┘
                  ↓
   ┌──────────────────────────────┐
   │     DETERMINE END POINT 1    │─── S76
   └──────────────┬───────────────┘
                  ↓
   ┌──────────────────────────────┐
   │    MEASURE TIME BETWEEN START │─── S77
   │    POINT 1 AND END POINT 1    │
   └──────────────┬───────────────┘
                  ↓
   ┌──────────────────────────────┐
   │   MOVE CALIPER TO POSITION OF │─── S78
   │   START POINT 2 BY TRACK BALL │
   └──────────────┬───────────────┘
                  ↓
   ┌──────────────────────────────┐
   │     DETERMINE START POINT 2  │─── S79
   └──────────────┬───────────────┘
                  ↓
   ┌──────────────────────────────┐
   │   MOVE CALIPER TO POSITION OF │─── S80
   │    END POINT 2 BY TRACK BALL  │
   └──────────────┬───────────────┘
                  ↓
   ┌──────────────────────────────┐
   │     DETERMINE END POINT 2    │─── S81
   └──────────────┬───────────────┘
                  ↓
   ┌──────────────────────────────┐
   │    MEASURE TIME BETWEEN START │─── S82
   │    POINT 2 AND END POINT 2    │
   └──────────────┬───────────────┘
                  ↓
          ┌─────────────┐
          │     END     │
          └─────────────┘
```

## FIG.8

FIG.9

FIG.10

(a)

COMPARISON OF PIXEL BRIGHTNESS IN THE
NEIGHBORHOOD OF UPPER WALL OF BLOOD VESSEL

Pix_(n)                                          Pix_(k)

255

Pix_(n+△)

BRIGHTNESS VALUE

UPPER
WALL OF
BLOOD
VESSEL

INNER MEMBRANE
OF BLOOD VESSEL

Pix_(n+△+1)

0
 0                                                   A
Pix_(n+1)

(b)

COMPARISON OF PIXEL BRIGHTNESS IN THE
NEIGHBORHOOD OF LOWER WALL OF BLOOD VESSEL

Pix_(q+□+1)

255

BRIGHTNESS VALUE

Pix_(q+1)

LOWER
WALL OF
BLOOD
VESSEL

INNER MEMBRANE
OF BLOOD VESSEL

Pix_(q)

Pix_(q+□)

0
 0                                                   A

## FIG.11

11

| 11a | 11b | 11c |
|---|---|---|
| TISSUE DISPLACEMENT AMOUNT CALCULATOR | ELASTICITY DISTORTION CALCULATOR | ELASTIC DATA ANALYZER |

ELASTICITY CALCULATING MEANS

## FIG.12

15

15b

15e    15c    15d    15a

FIG.13

**10**

**10a**                                    **10b**

DATA                           · DATA

FRAME
MEMORY                        FRAME                DATA
CONTROLLER      CONTROL        MEMORY
                SIGNAL

READ-OUT
SIGNAL

SAVING MEANS

## FIG.14

HEAD
↑

3

(a)

○

↓ FOOT

TOP VIEW

MOVEMENT
CAUSED BY
BREATHING
MOTION

3

←
HEAD

○

→
FOOT

SIDE VIEW

3

(b)

⇒

⇒

PRESS AMOUNT: LARGE

PRESS AMOUNT: SMALL

PRESS AMOUNT: SMALL

→ TIME

EXPIRING
STAGE

INSPIRING
STAGE

EXPIRING
STAGE

(c)

DISPLACEMENT

51B 51A 51B 51C 51B 51A 51B

0

→ TIME

INSPIRING
STAGE

EXPIRING
STAGE

INSPIRING
STAGE

EXPIRING
STAGE

RELATIONSHIP BETWEEN BREATHING MOTION AND
DISPLACEMENT OF TISSUE (LIVER)

# FIG.15

## (a)

11

TISSUE DISPLACMENT AMOUNT CALCULATOR — 11a

DISPLACEMENT ANALYZER — 11d

ELASTICITY DISTORTION CALCULATOR — 11b

ELASTICITY DATA ANALYZER — 11c

ELASTICITY CALCULATING MEANS

## (b)

SMALL DISPLACEMENT AMOUNT PERIOD

... | N FRAME | N+1 FRAME | ...

11a — [ N | N+1 ]

11d — DISPLACEMENT AVERAGE VALUE ≥ K

11b — 

LARGE DISPLACEMENT AMOUNT PERIOD

| N FRAME | N+1 FRAME | N+2 FRAME | ...

11a — [ N | N+1 ]

11d — DISPLACEMENT AVERAGE VALUE < K

## INTERNATIONAL SEARCH REPORT

| | International application No. |
|---|---|
| | PCT/JP2006/311267 |

| A. CLASSIFICATION OF SUBJECT MATTER |
|---|
| *A61B8/08*(2006.01)i |
| According to International Patent Classification (IPC) or to both national classification and IPC |

| B. FIELDS SEARCHED |
|---|
| Minimum documentation searched (classification system followed by classification symbols) |
| A61B8/08 |
| Documentation searched other than minimum documentation to the extent that such documents are included in the fields searched |
| Jitsuyo Shinan Koho     1922-1996   Jitsuyo Shinan Toroku Koho   1996-2006 |
| Kokai Jitsuyo Shinan Koho   1971-2006   Toroku Jitsuyo Shinan Koho   1994-2006 |
| Electronic data base consulted during the international search (name of data base and, where practicable, search terms used) |
| WPI |

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2005-58533 A (Matsushita Electric Industrial Co., Ltd.), 10 March, 2005 (10.03.05), (Family: none) | 1-17 |
| A | JP 5-317313 A (Ken ISHIHARA, Hitachi Medical Corp.), 03 December, 1993 (03.12.93), (Family: none) | 1-17 |
| A | JP 2002-209857 A (GE Medical Systems Global Technology Co., LLC), 30 July, 2002 (30.07.02), (Family: none) | 1-17 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 August, 2006 (21.08.06) | 29 August, 2006 (29.08.06) |
| Name and mailing address of the ISA/ | Authorized officer |
| Japanese Patent Office | |
| Facsimile No. | Telephone No. |

Form PCT/ISA/210 (second sheet) (April 2005)

**EP 1 891 900 A1**

| INTERNATIONAL SEARCH REPORT | International application No. |
| --- | --- |
| | PCT/JP2006/311267 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2004/103185 A1  (Matsushita Electric Industrial Co., Ltd.), 02 December, 2004 (02.12.04), (Family: none) | 1-17 |
| A | US 5846202 A  (ACUSON CORP.), 08 December, 1998 (08.12.98), (Family: none) | 1-17 |

Form PCT/ISA/210 (continuation of second sheet) (April 2005)

30

<table>
<tr><td>**INTERNATIONAL SEARCH REPORT**</td><td>International application No.<br>**PCT/JP2006/311267**</td></tr>
</table>

**Box No. II   Observations where certain claims were found unsearchable (Continuation of item 2 of first sheet)**

This international search report has not been established in respect of certain claims under Article 17(2)(a) for the following reasons:

1. ☒ Claims Nos.: 18-20
   because they relate to subject matter not required to be searched by this Authority, namely:
   The inventions as set forth in the above claims are deemed to be concerned with diagnostic methods to be practiced on the human body.  Consequently, the inventions as set forth in the above claims relate to a subject matter which this International Searching Authority    (continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.

PCT/JP2006/311267

Continuation of Box No.II-1 of continuation of first sheet(2)

is not required, under the provisions of Article 17(2)(a)(i) of the PCT and Rule 39.1(iv) of the Regulations under the PCT, to search.

Form PCT/ISA/210 (extra sheet) (April 2005)

**EP 1 891 900 A1**

## REFERENCES CITED IN THE DESCRIPTION

### Patent documents cited in the description

- JP 2000229078 A